# EUROPEAN PATENT SPECIFICATION

(11) **EP 2 288 321 B1**
(45) Date of publication and mention of the grant of the patent: **23.10.2019**
(21) Application number: 09755249.1
(22) Date of filing: 27.05.2009
(51) Int. Cl.: A61F 13/15, A61M 1/00

(54) **CONTROL UNIT WITH PUMP MODULE FOR A NEGATIVE PRESSURE WOUND THERAPY DEVICE**
KONTROLLEINHEIT MIT PUMPENMODUL FÜR EINE NEGATIVDRUCK-WUNDBEHANDLUNGSEINRICHTUNG
UNITÉ DE COMMANDE AVEC MODULE DE POMPE POUR UN DISPOSITIF DE TRAITEMENT DE PLAIE À PRESSION NÉGATIVE

(30) Priority: 27.05.2008 US 128942 P
(43) Date of publication of application: 02.03.2011
(73) Proprietor: Smith & Nephew, Inc., Memphis, TN 38116 (US)
(72) Inventor: BUAN, John, Maple Grove MN 55311 (US)
(74) Representative: Smith & Nephew
(86) International application number: PCT/US2009/003207
(87) International publication number: WO 2009/145886

(56) References cited:
- WO-A2-2008/036359
- US-A1- 2007 005 028
- US-A1- 2007 005 028
- US-A1- 2007 219 532
- US-A1- 2007 265 585
- US-A1- 2008 004 549

## Description

### RELATED APPLICATIONS

This application claims priority to United States Provisional Application 61/128,942 filed on May 27, 2008. The invention in the present application relates, generally, in subject matter to the devices disclosed in Applicant's own U.S. Patent Pre-Grant Publication Nos. 2007/0265585 and 2007/0265586.

### TECHNICAL FIELD

The present invention relates, in general, to a device with a pump for providing negative pressure for a wound therapy device that is capable of treating a variety of chronic and acute wound types, including, but not limited to, infection wounds, venous ulcers, arterial ulcers, diabetic ulcers, burn wounds, post amputation wounds, surgical wounds, and the like.

### BACKGROUND

Negative pressure therapy has been one method used for the treatment of a variety of wounds by practitioners in the art. Conventional negative pressure therapy devices are generally large in size and often require the use of complicated equipment such as suction pumps, vacuum pumps and complex electronic controllers.

Some of the currently available negative pressure wound therapy systems do not separate the expensive control and programming portions of the system from the mechanical pumping portion. Thus, in practice, the clinician must send the whole system for sterilization before using the device on a second, or subsequent, patient as both portions are potentially exposed to bacteria and wound exudates.

Specifically, most commercially available negative pressure wound therapy systems (with the exception of those available from Applicant and described below) are configured to allow exudates and liquid that are removed from the wound to pass outside the dressing to a canister or other structure for retaining the exudates and liquids. If this canister or other structure is incorporated into the apparatus that provides the negative pressure (i.e., the pump), then the apparatus tends to be bulky and rather large and can restrict the movement and mobility of a patient being treated.

In addition, the conventional apparatuses that provide the negative pressure for typical wound therapy devices (e.g., dressings) require a substantial power source such as a constant access to an outlet, or bulky, rechargeable batteries. This is a result of the power needed to run the pump because of the leaking of the dressing that causes dissipation of the negative pressure. Additionally, removal of wound fluid from the dressing requires the pump to be run continuously to maintain negative pressure within the dressing.

Newer wound therapy dressings, such as those described in Applicant's own U.S. Patent Pre-Grant Publication Nos. 2007/0265585 and 2007/0265586, are directed towards retaining the exudates and liquids within the wound therapy dressing itself.

In addition, some of such newer wound therapy dressings are more efficient and less likely to leak or allow the negative pressure to dissipate. For example, in Applicant's own U.S. Provisional Application No. 61/128,957, such a newer dressing is disclosed. US2007/0219532 A1 discloses a device for wound drainage and treatment with suction using a portable pump unit that is configured to produce controlled levels of negative pressure in a continuous or intermittent mode. US2007/0005028 A1 relates to a wound treatment apparatus for use with vacuum bandages of the type that dispenses fluid to a wound and draws fluid away from the wound. WO2008/036359 A2 relates to tissue treatment systems and in particular to a component module for a reduced pressure treatment system.

Thus, it would be beneficial to provide an apparatus that provides negative pressure that does not include a canister and does not require a large power source.

In addition, it would be beneficial to provide a device which will allow for a device in which the pump unit may be disposable. Thus, the possibility of cross-contamination between patients is lessened.

### SUMMARY OF THE INVENTION

The invention is defined by claim 1. Preferred embodiments are defined in the dependent claims. In accordance with an embodiment of the invention, the invention is a device with a control unit module and a pump module. The control unit module can functionally provide power to the entire system, can allow the user to program the device for therapy and can display relevant operational information including current settings and error messages. The pump module connects at least electrically and mechanically, but not fluidically, with the control unit module and contains the vacuum pump and other support systems. In this way the control unit module is isolated from the functional system plumbing and is unlikely to come in contact with wound exudates and/or bacteria through the pressure hoses that connect to the negative pressure wound dressing. Thus, the control unit module would not need aggressive cleaning when moved to another patient.

The invention is an apparatus as defined in claim 1 to be used to provide negative pressure for a wound therapy dressing. The apparatus includes a pump for creating a negative pressure and may include a port for providing communication of the negative pressure, and, a power source for the pump, wherein the power source comprises, for example, three AA batteries.

The apparatus may include a housing containing the pump, the port and the power source, wherein the housing has a handheld size.

Also disclosed is a method for treating a wound with negative pressure that does not form part of the invention. The method generally may include the steps of providing a wound therapy dressing, providing an apparatus including a pump contained in a housing, wherein the housing has a handheld size, and, utilizing the pump to provide a negative pressure, wherein the exudates are retained within the wound therapy dressing.

As will be described in detail below, certain aspects of some of the embodiments of the present invention provide an apparatus including a pump that is handheld. This is believed to be a result of at least two separate advances in the design of negative pressure wound therapy devices.

First, due to an increase in the efficiency of some negative pressure wound therapy dressings, the pumps that provide negative pressure to such dressings do not need such a large power source. It would not have been economically feasible to use commercial disposable (or rechargeable) batteries such as AA, AAA, and the like. The commercial disposable (or rechargeable) batteries would have such a high drain rate, that they would need to be changed constantly. However, because the efficiency of negative pressure wound therapy dressings is increasing, the pump does not require as large of a power source. Accordingly, an apparatus with a pump can be constructed that is handheld, and, that is substantially smaller than conventional pump apparatuses.

Second, due to the types of negative pressure wound therapy dressings wherein exudates are retained within the housing of the wound therapy dressing, the apparatus no longer requires a container or other large structure to collect and retain the exudates and liquid. Thus, the apparatus can be made smaller because it does not require a structure to collect and retain the exudates and liquid.

Thus, based upon the above and other aspects of the invention, the present invention provides one or more embodiments that are handheld. The benefits of having a handheld apparatus include increasing the mobility and movement of a patient being treated with negative pressure wound therapy. A patient could mount such a device to his or her belt and then move around in a relatively normal fashion. In addition, in general, having a smaller apparatus would be beneficial in hospital where storage space may be at a premium.

### BRIEF DESCRIPTION OF THE DRAWINGS

The present invention will become more fully apparent from the following description and appended claims, taken in conjunction with the accompanying drawings. Understanding that the accompanying drawings depict only typical embodiments, and are, therefore, not to be considered to be limiting of the scope of the present disclosure, the embodiments will be described and explained with specificity and detail in reference to the accompanying drawings as provided below.
Figure 1 is a front view of an apparatus according to the present invention.
Figure 2 is a back view of an apparatus according to the present invention.
Figure 3 is a view of a pump module according to the present invention.
Figure 4 is another view of a pump module according to the present invention.
Figure 5 is a view of a control unit module according to the present invention.
Figure 6 is another view of a control unit module according to the present invention.
Figure 7 is a front perspective view of a belt case to hold an apparatus according to the present invention.
Figure 8 is a back perspective view of a belt case to hold an apparatus according to the present invention.

### DETAILED DESCRIPTION OF THE PRESENT INVENTION

It will be readily understood that the components of the embodiments as generally described and illustrated in the Figures herein could be arranged and designed in a wide variety of different configurations. Thus, the following more detailed description of various embodiments, as represented in the Figures, is not intended to limit the scope of the present disclosure, but is merely representative of various embodiments. While the various aspects of the embodiments are presented in drawings, the drawings are not necessarily drawn to scale unless specifically indicated.

The described embodiments are to be considered in all respects only as illustrative and not restrictive. The scope of the invention is, therefore, indicated by the appended claims rather than by the foregoing description.

Reference throughout this specification to features, advantages, or similar language does not imply that all of the features and advantages that may be realized with the present invention should be or are in any single embodiment of the invention. Rather, language referring to the features and advantages is understood to mean that a specific feature, advantage, or characteristic described in connection with an embodiment is included in at least one embodiment of the present invention. Thus, discussion of the features and advantages, and similar language, throughout this specification may, but do not necessarily, refer to the same embodiment.

Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

In the following description, numerous specific details are provided, such as examples of housings, barriers, chambers etc., to provide a thorough understanding of embodiments of the invention. One skilled in the relevant art will recognize, however, that the invention can be practiced without one or more of the specific details, or with other methods, components, materials, and so forth. In other instances, well-known structures, materials, or operations such as vacuum sources are not shown or described in detail to avoid obscuring aspects of the invention.

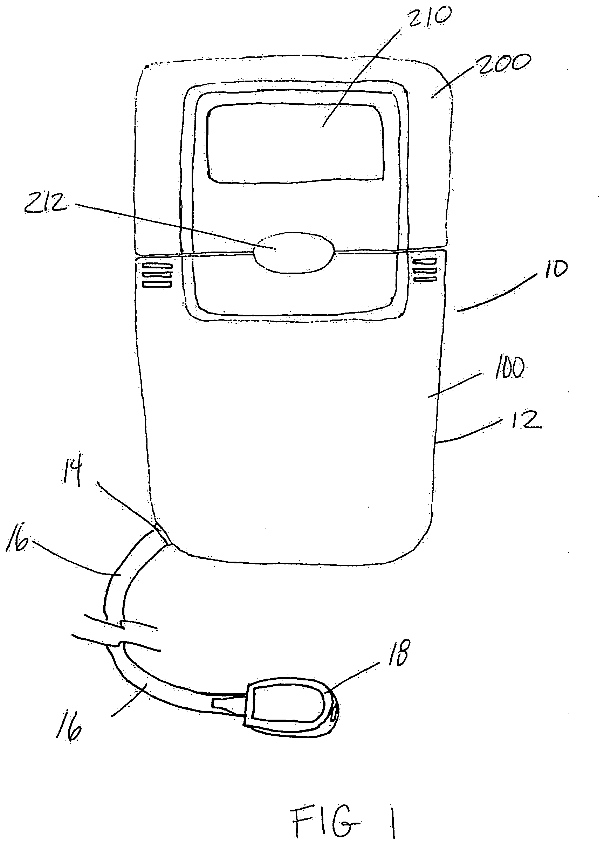

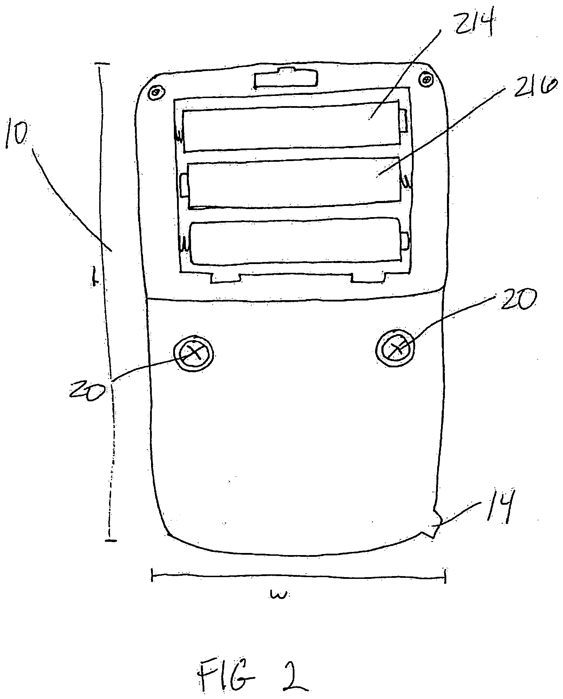

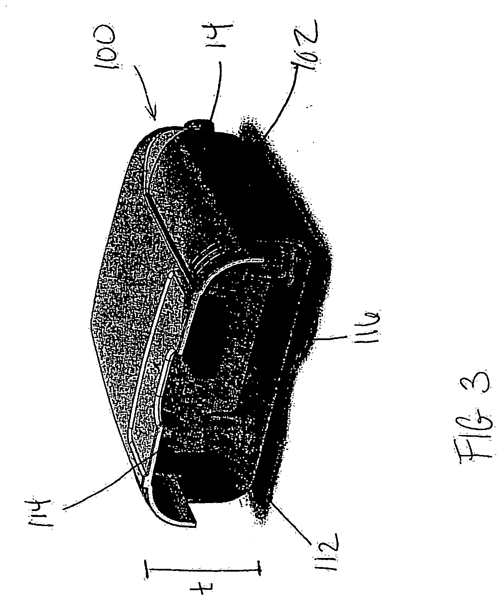

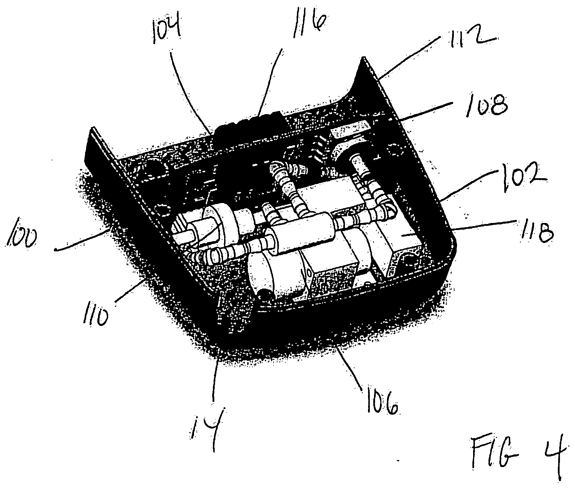

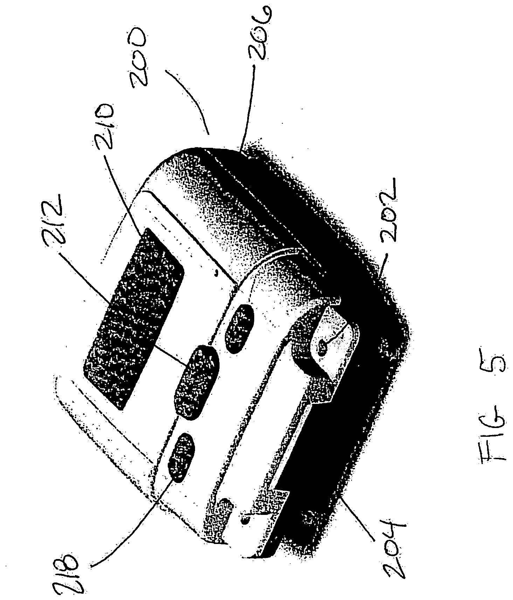

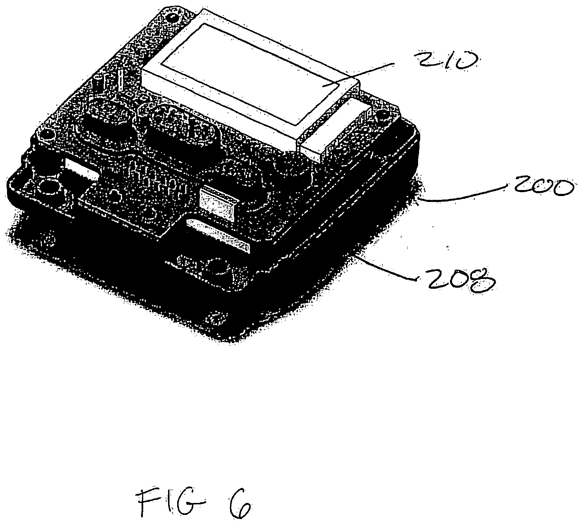

Referring now to Figures 1 through 6, an apparatus 10, according to one embodiment of the present invention, generally includes a pump module 100 and a control unit module 200. The apparatus 10 may include a housing 12, or alternatively the pump module 100 may include a housing 102 and the control unit module 200 may include a housing 206. It is preferred that the housing 12 (or alternately the housing 102 and housing 206) is handheld. As used herein, "handheld" is meant to mean that the housing 12 of the apparatus is generally smaller than conventional devices, and, has a length (I) less than approximately 22,86 cm (9 inches) has a width (w) less than approximately 17,78 cm (seven inches) and has a thickness (t) less than approximately 10,16 cm (four inches). In a preferred embodiment, the apparatus has a length (I) of less than or equal to 12,7 cm (five inches), a width (w) of less than or equal to 7,62cm (3 inches) and a thickness (t) of less than or equal to 3,81 cm (an inch and a half). In addition, a preferred embodiment of the apparatus weighs approximately 227 g (eight ounces).

The apparatus 10 may also include a port 14. The port 14 may further include tubing 16 and a quick release valve 18. As used herein "quick release valve" is intended to mean a valve or connection point that will allow the apparatus 10 to communicate negative pressure to a negative pressure wound therapy dressing and that includes one or more structures (such as a button) to maintain the connection.

For example, a quick release valve may be a female connector that includes structure to obtain an interference fit with a male connector. The female connector may also include a button that communicates with the structure. When it is desired that the two connectors be disengaged, one need simply depress the button, which would move the structure and disengage the male connector. This description is merely exemplary of a typical quick release valve.

The pump module 100 generally may include a circuit board 104, a pump 106 and a pressure sensor 108. It is also contemplated that the pump module 100 includes a check valve 110 for preventing flow of positive pressure from the pump module 100 to the dressing.

The pump 106 is capable of operating at pressures typical of negative pressure wound therapy, *e.g.* -25 mmHg to -250 mmHg. Currently, three types of pumps are contemplated to be used with the present invention. A continuous pump is a pump that provides and maintains a desired negative pressure for wound therapy. An intermittent pump is a pump that provides a desired negative pressure for wound therapy and then ceases providing pressure while the negative pressure is released. A third pump, described herein as a "hybrid pump" is a pump capable of operating as either a continuous pump or as an intermittent pump.

In an embodiment with an intermittent pump, it is contemplated that the pump module 100 includes a solenoid valve 118 located between and communicating with the negative wound pressure dressing and the pump. In use, once the intermittent pump has reached the desired pressure, the solenoid valve 118 can open and allow atmospheric air into the negative wound pressure dressing.

In an embodiment with a continuous pump, the pressure sensor 108 can detect a leak in the negative wound pressure dressing and communicate with the pump 106 so that the pump 106 provides the required pressure.

The circuit board 104 can manage and control all electrical aspects of the pump module 100.

The pump module 100 may be removably attached to the control unit module 200. The pump module 100 can be removably attached through any number of mechanical structures, including, but not limited to, tabs, screws, interference members, buttons, clips, snaps, bolts, pins or any other structure that will selectively maintain a connection between the pump module 100 and control unit module 200. In the embodiment depicted screws 20 pass through apertures 112 in the pump module 100 and through a second set of apertures 202 and terminate in screw caps 114 in the pump module 100.

As explained above, the control unit module 200 is isolated, fluidically, from the pump module 100 and specifically from the functional system plumbing and, therefore is unlikely to come in contact with wound exudates and/or bacteria through the pressure hoses that connect to the negative pressure wound dressing. Thus, the control unit module 200 would not need aggressive sterilization when utilized with another patient.

The control unit 200 is also in electronic communication with the pump module 100. In an embodiment shown, the control unit 200 includes a female connection 204 and the pump module 100 includes a male connection 116. When the control unit 200 and pump module 100 are removably attached, the male connection 116 communicates to the female connection 204. Additionally, the electrical connector on the control unit module 200 is preferred to be of the female type to prevent human contact with the power source. Additionally, the electrical connectors, *e.g*., 204, 116, can also provide the mechanical connection between the pump module 100 and control unit module 200, described above.

The control unit module 200 may also include, generally, a second housing 206, a second circuit board 208, a display 210, a button 212 and a power source 214. The power source 214 can be, but is not limited to, an AA battery 216. Indeed, other conventionally obtained disposable batteries (and smaller rechargeable batteries) are likewise contemplated-including, for example, AAA, A, C, and D sized batteries.

The button 212 can be a power button (*i.e.,* ON/OFF button). Additional buttons can include programming and/or mode selection buttons 218. If the housing 102 of the pump module 100 includes a sliding portion, it is contemplated that the sliding portion, in a closed configuration, cover one of more of the programming and/or mode selection buttons 218. The user can manipulate the sliding portion to an open position to access the buttons 218.

The display 210 can be an LCD display or an LED display, or any other display.

The second circuit board 208 is capable of detecting the type of pump 106 that is located in the pump module 100. One method that can be used for detecting the type of pump involves the use of resistors. For example, the second circuit board 208 can be pre-programmed with a specific resistance value for each of the three types of pumps described above (continuous, intermittent, or a pump capable of both). When the pump module 100 is connected to the control unit module 200 (since they are in electrical communication), the second circuit board 208 can detect the resistance of the pump. The second circuit board 208 can then communicate with the pump 106 allowing the pump to function appropriately. Additionally if the pump is one that is capable of functioning as either a continuous pump or an intermittent pump, when the second circuit board 208 detects the pump, the control unit can give the user the option of selecting the type of pump therapy to be used. For example, the display may display a message, and the user can depress a button to select the type of pump therapy to use.

The above description relates to an apparatus. As previously mentioned the present disclosure is also directed to a method for treating a wound with negative pressure that does not form part of the invention.

Generally, this method includes the steps of providing a wound therapy dressing, providing an apparatus including a pump contained in a housing, wherein the housing has a handheld size, and, utilizing the pump to provide a negative pressure, wherein the exudates are retained within the wound therapy dressing. It is preferred that the apparatus be the one described herein. Moreover, it is preferred, but not required, that the wound therapy dressing be one described in Applicant's own U.S. Patent Pre-Grant Publication Nos. 2007/0265585 and 2007/0265586.

The disclosed method may also include the step of selecting the mode of operation of the pump. This step is intended, but not required, to be utilized when the pump is a hybrid pump.

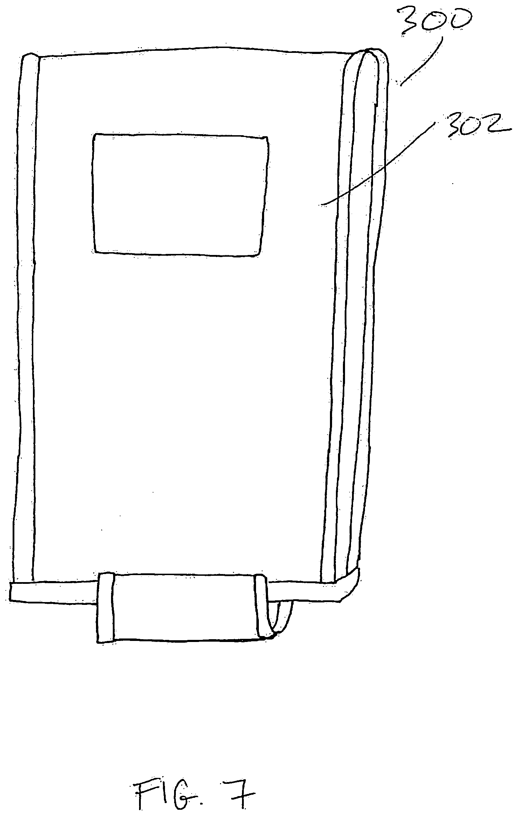

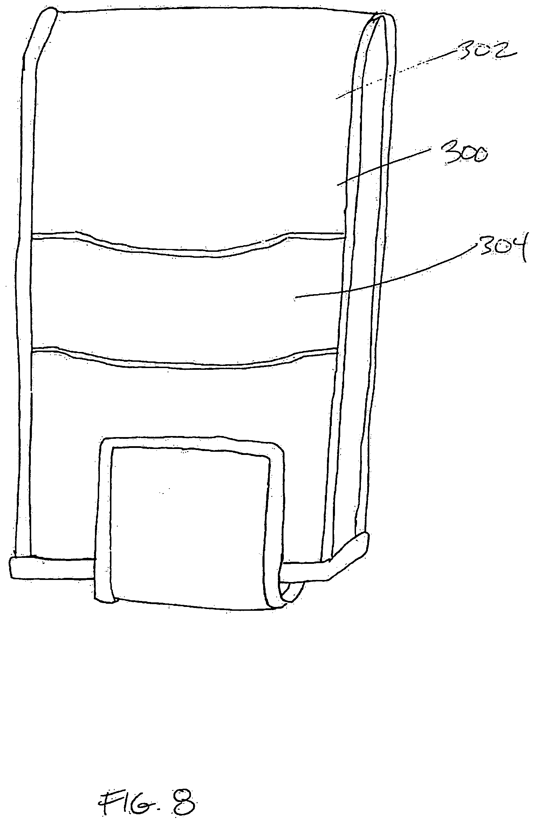

Furthermore, the disclosed method may include the step of mounting the apparatus to a belt. Figures 7 and 8 depict a means for mounting the apparatus to a belt 300. The means for mounting 300 can be, for example, a pouch 302 with a belt loop 304. Other means for mounting may include, loops, clips, snaps, cases, clasp, hooks, molded cases, hook and loop fasteners, button, zipper, magnet, and other similar structures.

Without further elaboration, it is believed that one skilled in the art can use the preceding description to utilize the present disclosure to its fullest extent. The examples and embodiments disclosed herein are to be construed as merely illustrative and not a limitation of the scope of the present disclosure in any way. The scope of the invention is defined by the following claims.

## Claims

1. An apparatus to be used to provide negative pressure for a wound therapy device, the apparatus comprising:
a pump module (100) comprising:
a pump (106) wherein the pump is of a type selected from the group consisting of: a continuous pump, an intermittent pump, and a hybrid pump; and,
a pressure sensor (108); and,
a control unit module (200) in electrical communication with the pump module, **characterised in that** the control unit module is capable of determining the type of pump located in the pump module.

2. The apparatus of claim 1 further comprising a port for communicating a negative pressure.

3. The apparatus of claim 2 wherein the port further comprises a tubing and a quick release valve.

4. The apparatus of claim 1 further comprising a power source.

5. The apparatus of claim 4 wherein the power source comprises at least two AA batteries.

6. The apparatus of claim 1 further comprising a housing wherein the housing has a handheld size.

7. The apparatus of claim 1 wherein the apparatus is configured to be used to provide negative pressure for a wound therapy dressing.

8. The apparatus of claim 7 wherein the pump is a continuous pump and wherein the pressure sensor is operable to detect a leak in the wound dressing and communicate with the pump so the pump provides the required pressure.

9. The apparatus of claim 7 or 8 wherein the pump is contained in a housing having a handheld size and wherein the apparatus does not include a canister,

## Patentansprüche

1. Eine Vorrichtung zur Verwendung, um Unterdruck für ein Wundtherapiegerät bereitzustellen, wobei die Vorrichtung Folgendes beinhaltet:
ein Pumpenmodul (100), beinhaltend:
eine Pumpe (106), wobei die Pumpe von einem Typ ist, ausgewählt aus der Gruppe, bestehend aus: einer Dauerpumpe, einer Intervallpumpe und einer Hybridpumpe; und
einen Drucksensor (108); und
ein Steuerungseinheitsmodul (200), das elektrisch mit dem Pumpenmodul kommuniziert, **dadurch gekennzeichnet, dass** das Steuerungseinheitsmodul in der Lage ist, den Typ der Pumpe, die sich in dem Pumpenmodul befindet, zu bestimmen.

2. Vorrichtung gemäß Anspruch 1, ferner beinhaltend einen Anschluss zum Kommunizieren eines Unterdrucks.

3. Vorrichtung gemäß Anspruch 2, wobei der Anschluss ferner einen Schlauch und ein Schnelltrennventil beinhaltet.

4. Vorrichtung gemäß Anspruch 1, ferner beinhaltend eine Energiequelle.

5. Vorrichtung gemäß Anspruch 4, wobei die Energiequelle mindestens zwei AA-Batterien (AA-Akkus) beinhaltet.

6. Vorrichtung gemäß Anspruch 1, ferner beinhaltend ein Gehäuse, wobei das Gehäuse eine handliche Größe aufweist.

7. Vorrichtung gemäß Anspruch 1, wobei die Vorrichtung zur Verwendung, um Unterdruck für einen Wundtherapieverband bereitzustellen, konfiguriert ist.

8. Vorrichtung gemäß Anspruch 7, wobei die Pumpe eine Dauerpumpe ist und wobei der Drucksensor betrieben werden kann, um ein Leck in dem Wundverband zu erkennen und mit der Pumpe zu kommunizieren, sodass die Pumpe den erforderlichen Druck bereitstellt.

9. Vorrichtung gemäß Anspruch 7 oder 8, wobei die Pumpe in einem Gehäuse enthalten ist, das eine handliche Größe aufweist, und wobei die Vorrichtung keinen Behälter umfasst.

## Revendications

1. Un appareil à utiliser afin de fournir une pression négative pour un dispositif de thérapie de plaie, l'appareil comprenant :
un module de pompe (100) comprenant :
une pompe (106), la pompe étant d'un type sélectionné dans le groupe constitué :
d'une pompe continue, d'une pompe intermittente, et d'une pompe hybride ; et,
un capteur de pression (108) ; et,
un module d'unité de commande (200) en communication électrique avec le module de pompe, **caractérisé en ce que** le module d'unité de commande est capable de déterminer le type de pompe situé dans le module de pompe.

2. L'appareil de la revendication 1 comprenant en outre un orifice pour communiquer une pression négative.

3. L'appareil de la revendication 2 dans lequel l'orifice comprend en outre une tubulure et une valve à libération rapide.

4. L'appareil de la revendication 1 comprenant en outre une source d'alimentation.

5. L'appareil de la revendication 4 dans lequel la source d'alimentation comprend au moins deux piles AA.

6. L'appareil de la revendication 1 comprenant en outre un boîtier, le boîtier ayant une taille portative.

7. L'appareil de la revendication 1, l'appareil étant configuré afin d'être utilisé pour fournir une pression négative pour un pansement de thérapie de plaie.

8. L'appareil de la revendication 7 dans lequel la pompe est une pompe continue et dans lequel le capteur de pression permet de détecter une fuite dans le pansement de plaie et de communiquer avec la pompe de façon à ce que la pompe fournisse la pression requise.

9. L'appareil de la revendication 7 ou de la revendication 8 dans lequel la pompe est contenue dans un boîtier ayant une taille portative et l'appareil n'incluant pas de récipient.
